## Europäisches Patentamt

## European Patent Office

## Office européen des brevets

(19)

(11) Publication number: **0 260 358**
**B1**

# EUROPEAN PATENT SPECIFICATION

(12)

(45) Date of publication of the patent specification:
**11.04.90**

(21) Application number: **86307203.9**

(22) Date of filing: **18.09.86**

(51) Int. Cl.⁴: **C12N 1/20**, A61K 39/02,
G01N 33/569, C07K 3/02,
C12P 21/00
// C12R1:35

(54) Specific mycoplasma membrane antigen and antibody and their clinical applications.

(43) Date of publication of application:
**23.03.88 Bulletin 88/12**

(45) Publication of the grant of the patent:
**11.04.90 Bulletin 90/15**

(84) Designated Contracting States:
**AT BE CH DE FR GB IT LI LU NL SE**

(56) References cited:
SCIENCE, vol.216, 16 April 1982, AAAS; P.C.HU et al.:
"Mycoplasma pneumoniae infection: Role of a surface
protein in the attachment organelle", pp. 313-315
CHEMICAL ABSTRACTS, vol. 103, no. 19, 11 Nov 1985,
Columbus, OH (US); P.C.HU et al.: "Demonstration of
multiple antigenic determinants on Mycoplasma
pneumoniae attachment protein by monoclonal
antibodies", p. 548, no. 158721r
CHEMICAL ABSTRACTS, vol. 104, no. 3, 20 Jan 1986,
Columbus, OH (US); G.TIGYI et al.: "Screening for
monoclonal antibodiesagainst a mycoplasmal membra
ne protein by protein electroblotting", p. 377, no. 18353t
CHEMICAL ABSTRACTS, vol. 105, no. 15, 13 Oct 1986,
Columbus, OH (US); J.MORRISON-PLUMMER et al.:
"Biological effects of anti-lipid and anti-protein
monoclonal antibodies on mycoplasma pneumoniae",
p. 492, no. 131798a
CHEMICAL ABSTRACTS, vol. 105, no. 19, 10 Nov 1986,
Columbus, OH (US); R.D.MADSEN et al.:

(73) Proprietor: **Lee, Sin Hang, 53, Milan Road, Woodbridge,
Connecticut(US)**

(72) Inventor: **Lee, Sin Hang, 53, Milan Road, Woodbridge,
Connecticut(US)**

(74) Representative: **Holmes, Michael John et al, Frank B.
Dehn & Co. European Patent Attorneys Imperial
House 15-19 Kingsway, London, WC2B 6UZ,(GB)**

(56) References cited: (continuation)
"Species-specific monoclonal antibody to
a 43,000-molecular-weight membrane protein of
mycoplasma pneumoniae", p.563, no. 170273v

Note: Within nine months from the publication of the mention of the grant of the European patent, any person may give notice to
the European Patent Office of opposition to the European patent granted. Notice of opposition shall be filed in a written reasoned
statement. It shall not be deemed to have been filed until the opposition fee has been paid (Art. 99(1) European patent
convention).

## Description

Mycoplasmas are the smallest free-living microorganisms known to man. They are smaller than bacteria and larger than most viruses, measuring about 10 x 200 nm in size. Because they lack a rigid cell wall, are very pleomorphic and stain poorly with dyes, the individual organisms are very difficult to recognize with light microscopy. In fact, the species Mycoplasma pneumoniae (M. pneumoniae), a pathogen causing human primary atypical pneumonia and upper respiratory tract infections, was initially given such ill-defined names as Eaton agent and pleuropneumonia-like organism (PPLO). Cultivation of the organism in cell-free media was not achieved until early 1960s. Methods for isolation, propagation and specific identification of M. pneumoniae from patients' excretions remain highly technical and are performed routinely only in a few laboratories. Most clinical laboratories depend on either nonspecific serologic tests, such as cold agglutinin and MG streptococcus tests, or the complement fixation test for establishing diagnosis of M. pneumoniae infections in humans. [Textbook of Medicine, by P. B. Beeson and W. McDermott, W. B. Saunders Co., Philadelphia, 1975, pp. 270-274. Medical Microbiology and Infectious Diseases, by A. I. Braude, W. B. Saunders Co., Philadelphia, 1981, pp. 925-929. Respiratory Infections: Diagnosis and Management, by J. E. Pennington, Raven Press, New York, 1983 pp. 251-257.]

Several investigators have attempted to use an indirect fluorescent antibody technique, using M. pneumoniae grown in solid or liquid media as antigens. In 1962 Chanock, Hayflick and Barile reported successful transferring of mycoplasmal colonies from solid agar media to glass slides and demonstrating specific antibodies against these colonies in convalescent serum samples of patients recovering from M. pneumoniae infection. [Chanock, R.M., Hayflick, L. and Barile, M. F.; Proc. Nat. Acad. Science, 48:41-49, 1962.] However, because the colonies of the microorganisms are largely embedded in the agar and not readily removed by mechanical scraping, the researchers had to place the agar medium with grown colonies on the slide, then melt the agar away by raising its temperature to at least 80° to 85°C in order to remove the agar. Unfortunately, by doing so the antigen is partially denatured, and the technique cannot be used to produce a large number of slides with adherent colonies for diagnostic purpose in a clinical laboratory.

In 1978, Silis and Andrews suggested using the granular deposits from a liquid culture of M. pneumoniae as antigen for the indirect fluorescent antibody test instead of colonies grown in a solid medium so that the heating procedure can be omitted. These granular deposits were presumably mycoplasmal colonies. [Silis, M. and Andrews, B.E.: A simple test for Mycoplasma pneumoniae IgM. Zentralblatt für Bakteriologie, I. Abteilung Original, 241:239-240, 1978.] However, this approach has not been widely used because antigenic specificity of these deposits or whole colonies has not been proven. The original abstract of these authors does not contain any technical details regarding how to prepare the antigen. Generally the technique used is acetone fixation, as illustrated by two recent articles which made reference to the details of methodology [Buck, D.W., Kennett, R.H., and McGarrity, G.: Monoclonal antibodies specific for cell culture mycoplasmas, in vitro, 18:377-381, 1982. Lind, K., Lindhardt, B., Schütten, H. J., Blom, J. and Christiansen, C.: Serological Cross-Reactions between Mycoplasma Genitalium and Mycoplasma Pneumoniae, J. Clinical Microbiology 20:1036-1043, 1984.] A similar acetone-fixed antigen has been used for the detection of antibody to Mycoplasma genitalium, an organism causing non-gonorrheal urethritis (Furr, P. M., and Taylor-Robinson, D.: Microimmu nofluorescence technique for detection of antibody to Mycoplasma genitalium. J. Clin. Path. 37:1072-1074, 1984). None of these authors have made any claim that the microcolonies grown in the liquid media yield any results that are different from those grown in solid media as reported by Chanock, Hayflick and Barile (see above reference Proc. Nat. Acad. Science 48:41-49, 1962). In the liquid media, only a small percentage of the mycoplasma organisms grow in the form of spherules or microcolonies (Boatman, E. S. and Kenny, G.: Morphology and ultrastructure of Mycoplasma pneumoniae spherules. J. Bacteriology 106:1005-1015, 1971). The cost of mass-producing these microcolony antigens for use in the clinical laboratories is prohibitive.

Another approach to procure a large quantity of M. pneumoniae antigen for indirect fluorescent antibody test in a clinical laboratory is to use a commercial M. pneumoniae preparation which has been grown in a liquid medium, centrifuged and freeze-dried, and marketed by Wellcome Laboratories, Research Triangle Park, N.C., U.S.C., as reported by Carter and Carter. [Carter, J. B. and Carter, S. L.: Acute-Phase, Indirect Fluorescent Antibody Procedure for Diagnosis of Mycoplasma Pneumoniae Infection. Annals of Clinical and Laboratory Science, 13:150-155, 1983.] Unfortunately, by using this material as antigen, the amounts reported that "a positive result appears as an applegreen, fluorescent slurry of particulate matter consistent with the almost submicroscopic morphology of the M. Pneumoniae organism" (p. 152). In other words, the authors found it difficult to distinguish M. pneumoniae organisms from other particulate matters from the liquid media with confidence. It has been well-recognized by workers in the field of mycoplasma research that precipitates other than mycoplasma organisms invariably form in the agitated liquid media during incubation. The precipitates are not readily distinguishable from the higher pleomorphic minute microorganisms. Serum samples containing high titer of non-specific immunoglobulin M. such as rheumatoid factors, may give rise to false positive results. [MP-1-IgM. Zeus™ Technologies, Inc., Series No. 17000 M. 1984 (see "Limitations", No. 6), P.O. Box 177, Raritan, N.J. 08869.]

From these earlier publications by others, it has become clear that the only reliable morphologic criterion for recognizing the M. pneumoniae organisms is by its colony-forming characteristic, i.e., to work with concentrated, purified microcolonies. However, the whole colonies are difficult to produce in a large quantity and may not contain abundant species-specific antigens which can be distinguished morphologically from non-specific antigens.

It is an object of the present invention to provide mycoplasma in the form of medusoid microcolonies and a method for selective propagation of these in dialysed, particle-free liquid media by "weeding out" the single growing units through a series of subcultures.

It is another object of the invention to provide a method for making an inoculum by breaking up of the tuberous floating colonies of mycoplasma by gentle homogenization into small fragments, but not into single growing units. The inoculum is used as seed for the last culture in which each fragment grows into a medusoid in 4–5 days.

Another object of the invention is the identification of specific membrane antigen.

It is a further object of the invention to use aldehydes, desirably glutaraldehyde to stabilize the specific membrane antigens of mycoplasma.

The present invention provides a liquid medium grown mycoplasma predominantly in the form of medusoid microcolonies comprising polyps or vesicles 1–10 μm in diameter against which antibodies to one or more specific membrane antigens of said mycoplasma can be raised.

The present invention also provides a method to produce and stabilize large quantities of actively growing mycoplasma organisms in microcolonies or medusoids which can be identified microscopically with ease and have a high content of species-specific membrane antigen. The specific membrane antigen can be used to detect specific antibodies against mycoplasma in patients' serum samples. It can also be used to elicit antibody production in animals so that the specific membrane antigen of mycoplasma can be detected using the animal antibodies as tracers, in the sputum or in other excreta of patients having a mycoplasma infection.

The present invention is related to a test using a technique for massive propagation of mycoplasma colonies with a species-specific membrane antigen, and an approach enabling the recognition of a specific antigen-antibody reactions with these antigens or antibodies.

The importance of an immunofluorescence method for detection of mycoplasma organisms and their antibody is emphasized by its role in establishing the causative relationship between Mycoplasma pneumoniae and primary atypical pneumonia in humans. [Liu, C.: J. Experimental Medicine 106:455-466, 1957.] The test appears to be species specific under optimum conditions in the hands of experts.

Acetone, a common solvent fixature for preparation of immunofluorescence antigens, tends to remove the specific membrane antigen from these peripheral cells. In order to preserve the membrane antigen on glass slides, the mycoplasma medusoids must be fixed by non-solvent fixatives, such as aldehydes. The aldehyde employed is not critical for operative results; glutaraldehyde is presently the aldehyde of choice. Other aliphatic aldehydes may be used such as formaldehyde, paraformaldehyde and dialdehydes such as adipaldehyde. Using these aldehyde- fixed medusoids as antigen, immunoglobulins M and G antibodies specific for M. pneumoniae membrane can be detected in the serum samples of patients suffering from M. pneumoniae infection or in immune serum of rabbits immunized with M. pneumoniae medusoids. Aldehydes, especially glutaraldehyde, inhibit the non-specific somatic antigen often observed in the old colonies.

Other investigators have used organic solvents, in particular acetone, as fixatives to inactivate and preserve the mycoplasma antigen either in the form of whole colonies (granular deposits) or as slurry. The present invention is in part developed from the recognition that this traditional approach has at least two conceptional and technical flaws.

Firstly, some important components, such as the glycolipids in the mycoplasma membrane, would be removed by the organic solvents. Structurally located at the surface of the microorganism, the membrane antigens must play the most important role in the initial interaction with the susceptible cells of a host. Removal of the glycolipids invariably disrupts the integrity of the antigenic determinants of the membrane. A major antigen of M. pneumoniae, as measured by both rabbit immune and human convalescent serum, has been found in the lipid fraction of the organisms. [Kenny, G. E. and Gryston, J. T., J. Immunol. 95:19-25, 1965, especially pp. 23-24].

Secondly, the use of whole colonies or culture slurry as the antigen fails to take into consideration the age of the mycoplasma microorganisms. The process of aging in mycoplasma culture is known to be accompanied by a marked decrease in the activity of membrane-associated enzymes and by an increase in the protein-lipid ratio in the membrane fraction isolated from the osmotically-lysed cell suspensions, suggesting that the young organisms must contain physiologically more active and more lipid-rich membranes than the older cells [The Mycoplasmas - Cell Biology, Ed. M. F. Barile and S. Razin, Academic Press New York, 1979, pp. 315-319.] Because of the long replication time, each colony or granule recovered from a mycoplasma culture (usually at least several days old) is invariably composed of organisms of various ages. The youngest cells in logarithmic phase of replication are logically the most valuab le organisms to provide the most specific membrane antigen.

This new technology is based on the discovery that mycoplasma, such as M. pneumoniae, can be selectively cultivated in the form of tuberous floating colonies, or in the form of medusoid microcolonies when fragments of the tuberous floating colonies are incubated in a fresh medium for about 5 days. A single growing unit cannot form a medusoid in this short time because of the slow growth rate of M. pneumoniae. In these selected cultures, individual or isolated growing units of the mycoplasma organisms are kept to a relatively small number so that their competition with the floating colonies or medusoids for nutrients or for other growth factors is minimized. The mycoplasma organisms grown in this manner can be recognized easily under the microscope because they present themselves in the form of characteristic colonies. Since the base of the media has been dialyzed, and the horse serum has been filtered, and the incubation is carried out without agitation, no particulate matters are formed in these cultures other than microorganisms.

Although the membranes of mycoplasma have been known to be the most important structure of the microorganism, prior approaches have used chemical analyses to study the membranes, their ingredients, and the antigenicity of their chemical ingredients. These earlier techniques ignore the fact, here recognized, that only the very young cell, i.e. of only a few hours to one-day-old, at the growing edge of a colony or a medusoid, contain the specific membrane antigen. Chemical analysis of the membrane fractions, isolated from a whole culture or from an entire colony population, cannot distinguish the specific membrane antigen from the non-specific components. The entirely new technique of this invention deals with the antigenicity of the intact membrane of the very young M. pneumoniae polyps or vesicles, polysaccharides, proteins and lipids included in their natural spatial relationship.

Specific M. pneumoniae membrane antibody has been produced by immunizing rabbits with medusoids of M. pneumoniae and the non-specific antibodies can be removed by absorption with acetone-fixed colonies harvested from old M. pneumoniae cultures. Using the absorbed specific antibody M. pneumoniae membrane antigens in sputum can be detected either by an ELISA (enzyme-linked immunosorbent assay), [Lancet, August 21, 1976, pp. 406-407] or by an immunofluorescence technique.

The clinical application of this new technology can improve the accuracy in early diagnosis and early treatment of M. pneumoniae infection. Identification of specific membrane IgM antibody or observation of a rising titer of specific membrane IgG antibody in the patient's serum indicates that the patient is having or has just recovered from an acute active infection caused by M. pneumoniae. Serologic or immunofluorescent identification of the M. pneumoniae antigen or the characteristic membrane-delineated growing units in the sputum or sputum culture also indicates active proliferation of this microoganism in the upper respiratory tract, presumably evidence for an acute infection.

This invention further provides a basis for the development of monoclonal antibodies specifically against mycoplasma membrane antigens, using existing hybridoma techniques. In this case, the glutaraldehyde fixed medusoids provided here can be used as the antigen for selecting the hybridoma cell lines that produce the specific monoclonal antibody against the membrane of the polyps or vesicles.

Because Mycoplasma pneumoniae and Mycoplasma genitalium share many common antigens, including those in the membranes of the medusoid colonies, the same antigen recommended for detecting antibodies in patients of M. pneumoniae infection can also be used for antibodies of M. genitalium, an organism causing non-gonorrheal urethritis. The specific anti-membrane antibodies raised by M. pneumoniae can also be used to detect the specific membrane of M. genitalium in the urethral exudates.

The invention will now be elucidated by means of the following examples which will illustrate the practices of the invention, without the same being limited thereto.

Example A

I. Preparation of Media.

1. The following ingredients are placed in a semipermeable tubing.

| | |
|---|---|
| Broth base, i.e. an infusion of beef heart muscle containing peptone such as the commercially available PPLO Broth Base (Difco Lab.) | 210 gm |
| Yeast extract, Yeastolate (Difco Lab.) | 30 gm |
| Thallium acetate | 412.5 mg |
| Distilled water | 1,000 ml |

2. The tubing is dialyzed in distilled water, total volume 10 liters including tubing contents, for three days at 2-5°C.

3. The tubing and contents within are then discarded. The volume of dialysate is then about 8,500 ml.

4. The dialysate is adjusted to a pH of about 7.8 with 1 N NaOH, and filtered through filter paper to remove any traces of particles.

5. Next, the dialysate is sterilized in an autoclave at 121°C for 20 minutes.

6. The final medium is then prepared so as to consist of 80 ml of dialysate, 20 ml of filtered gamma-globulin-free horse serum and 500 units of penicillin per ml, or any overall amount thereof in the same general proportion of the ingredients.

II. Selective Isolation of Tuberous Floating Colonies of M. Pneumoniae.

A stock culture of a standard strain of M. pneumoniae is inoculated in a test tube containing about 10 ml of liquid medium, and incubated at 37°C with a slow rotation for four days. About 0.5 ml of the initial culture is transferred to a second test tube with fresh medium at the end of the four-day incubation. About three serial subcultures in this manner will produce actively growing microoganisms of M. penumoniae in the liquid medium. After a four-day incubation in the last subculture, the liquid culture is left in the incubator without further rotation or agitation for 2-3 weeks. The sediment in the medium is sampled and observed for the appearance of tuberous floating colonies of M. pneumoniae of about 25-50 μm in diameter, or conglomerates of the colonies, microscopically. When their presence is confirmed, the sediment is re-suspended and the suspension is centrifuged at a speed of 1,500 r.p.m. for 20 minutes. The supernatant liquid is then replaced with fresh liquid medium, and the sediment is incubated further for about seven days while being mixed by inverting the test tube once a day. The supernatant liquid is replaced with fresh medium every seven days. After about 3 weeks, tuberous floating colonies of mycoplasma become the prevalent form of growth, many forming conglomerates of over 100 μm in diameter. They are then transferred into larger tissue culture flasks with flat bottoms for further propagation.

III. Formation of Medusoid Microcolonies in Liquid Media.

About 500 tuberous floating mycoplasma colonies which have been incubated in a fresh liquid medium for 4-5 days and harvested by centrifugation, are transferred to a Potter-Elvehjem glass tissue grinder containing about 2 ml of liquid medium, and homogenized with a Teflon pestle driven by a motor turning at 100-200 r.p.m. for no more than one minute. At the end of homogenization, all colonies should have been disintegrated; this can be confirmed microscopically. Then the homogenate is mixed with about 50 ml of fresh medium and incubated in tightly capped plastic Petri. dishes or plastic tissue culture flasks with flat bottom. The medium in the culture is adjusted to about 3 mm in height. After a 5-day incubation at 37°C, round or ovoid microcolonies most of them ranging from 5 to 25 μm in diameter, or conglomerates of the microcolonies, are formed in the liquid culture either floating freely or loosely attached to the bottom of the container. The attached colonies are gently rinsed off the bottom with phosphate-buffered saline, pH 7.4 (PBS). The microcolonies are then harvested by a low-speed centrifugation (about 2,500-3,000 rpm). High speed centrifugation, for example 11,000 rpm (15,000 xg), destroys the polypoid form of growth and causes loss of the membrane antigens at the surface of the medusoids (see below).

After three washes in PBS, drops of a heavy suspension of microcolonies are places on glass slides and dried at room temperature. They can now be used as unfixed antigen preparations or preferably used after fixation in 1% glutaraldehyde in PBS for 2 minutes. Since these microcolonies are composed principally of numerous tiny polyps or vesticles, 1-10 μm in size that are delineated by membranes of 0.1-02. μm thick after immunofluorescent staining, and since they almost invariably surround a closely packed core of apparently less active organisms (thus resembling small jellyfish), they are referred to here as medusoid microcolonies, or medusoids.

IV. Indirect Immunofluorescence Test for Specific Anti-mycoplasma Antibodies in Patient Serum.

The serum samples of patients to be tested for a mycoplasma infection are diluted serially with a 2-fold dilution method, with PBS to 1:10, 1:20, 1:40, 1:80, 1:160, etc. One drop of the respective diluted serum samples is first reacted with the mycoplasma medusoid microcolonies dried on the slides as antigen. After washing off the patient serum, the antigen is then covered with a fluorescenated anti-human IgG or IgM antibodies raised in animals (rabbit, goat, sheep, swine, etc.). After the unbound fluorescent antibodies are washed off, the slides are mounted in buffered glycerin. The thus prepared and reacted microcolonies are then examined with a fluorescence microscope to determine the membrane fluorescence characteristics of the microcolonies.

When a significant concentration of M. pneumoniae antibodies (IgG or IgM or both) is present in the patient serum, the membranes and only the membranes of these polyps or vesicles at the surface or the periphery of the medusoids will flouresce. The quantity of the antibodies is then reflected by the titer (dilution). The membranes of the medusoid reacted with normal or control serum samples do not fluoresce and are therefore not visible.

(a) Comparison with aged colony cultures

When the floating colonies are allowed to age in the culture for three weeks or longer, the young mycoplasma cells at the periphery can no longer be demonstrated by the immunofluorescence technique, indicating that the specific membrane antigen has largely disappeared. However, these old colonies are not dead because actively growing vesicular cells with specific membrane antigen may grow out at the surface of these colonies after they are re-incubated in fresh media for two to four days.

(b) Comparison with fixed culture after acetone immersion

The specific membrane antigen of the medusoid which has been fixed in glutaraldehyde can no longer be demonstrated after the antigen preparations are immersed in acetone for 2 minutes. Ethanol and chloroform have a similar effect as acetone.

V. Production of Antibodies.

In order to produce antibodies against the specific membrane antigen of M. pneumoniae, washed live M. pneumonmiae medsoid microcolonies of a 4-day culture are injected intravenously into rabbits at a 10-14 day interval. After six to seven injections, serum samples of the immunized rabbits are tested for antibodies against the specific membrane antigens, using the above-described immunofluorescence technique. The binding of the IgG and IgM antibodies to the membrane of the mycoplasma medusoid microcolonies is demonstrated by fluorescent goat anti-rabbit immunoglobulin antibody. The rabbit immunoglobulins are isolated by the standard ammonium sulfate technique. The nonspecific antibodies, which are directed against the cytoplasm and the inactive forms of the mycoplasma cells, are absorbed by washed acetone-fixed tuberous colonies of a 4-week-old culture. After absorption, the rabbit immunoglobulins are highly specific for the membrane antigen of M. pneumoniae, and can be used to detect the soluble or membrane antigens of M. pneumoniae in the sputa and other exudates.

One such example is illustrated as follows:

## Example B

### 1. Specimen preparation:

A thick smear of sputum of patients with M. pneumoniae infection or of sputum containing M. pneumoniae grown in the laboratory is allowed to dry on a watch glass at 37°C (in one hour). The dried smear is extracted by acetone (2-5 ml). The acetone extract is transferred to a glass test tube, and the solvent is evaporated at about 37°C by inserting the test tube in a heating block. One ml of PBS, pH 7.4, containing 1% bovine serum albumin (BSA) is added and mixed with the residue, and the test tube is shaken in a 37°C water bath for one hour to obtain the soluble lipid fraction the insoluble residue is discarded as sediments after centrifugation.

### 2. Preparation of antibody-coated test tubes or membrane filters.

The glutaraldehyde-treated test tube method (M. J. Barrett, et al., U.S. Patent 4,001,583, as quoted in Methods in Enzymology, Vol. 73, p. 234-235, 1981) is followed. With this method, the specific M. pneumoniae membrane antibody (rabbit immunoglobulins) is bound to the bottom of polypropylene test tubes. Alternatively, a commercially available protein-binding membrane, such as Pall Biodyne™ Immunoaffinity Membrane (Biotechnology Division, Pall Ultrafine Filtration Corporation, Glen Cove, NY 11542), can be used. The purpose is to make the antibody adhere to a solid surface while preserving its specific antigen-binding activity.

The following steps are next performed:

(a) Incubate the soluble lipid fraction, or the cell lysate*, of the sputum with the antibody immobilized on the plastic tube surface or on the membrane filter for 30 minutes at room temperature.

(b) Wash the test tube or membrane thoroughly with PBS.

(c) Add an enzyme-labelled rabbit M. pneumoniae specific antibody (see above) carrying an alkaline phophatase covalently bound to the immunoglobulin molecules (S. AVRAMEAS, Immunochemistry, Vol. 6, pp. 43-52, 1969) to the test tubes or membranes.

(d) After incubation for 30 minutes, the unbound phosphatase-labelled antibody is rinsed off with a Tris-HCl buffered saline, pH 7.4. The bound labelled antibody is quantificated with a standard Bodansky or other method for alkaline phosphatase. The presence of high alkaline phosphatase activity indicates a high titer of acetone-extractable M. pneumoniae antigen in the patient's sputum. The control specimens without M. pneumoniae should be negative.

*Alternately, a deep sputum on bronchial washing specimen is centrifuged in PBS to obtain the desquamated epithelial cells. The sediment is lysed in 0.1 ml carbonate-bicarbonate buffer (0.1 M pH 10). The pH of the sputum lysate is brought to about pH 7.2-7.4 with 0.1 N HCL. This represents an aqueous extract to the used in place of the lipid fraction.

## Example C

Another example of using the specific antibody to detect M. pneumoniae specific membrane antigen in sputum or other exudates directly or after incubation in a liquid medium, is to stain the sputum smear or the sediment of a liquid culture of sputum with an indirect immunofluorescence technique. In this method, the smear of sputum or the sediment of a liquid culture of sputum on a microscopic slide is first air dried and fixed with 1% glutaraldehyde in PBS at pH 7.4 for 2 minutes. The specific rabbit anti-M. pneumoniae immunoglobulins are first allowed to react with the smear, and the bound immunoglobulins are then visualized with a fluorescent goat anti-rabbit gamma-globulin antibody (by the methods described above). If a polyp or vesicular microorganism of 1-10 μm in diameter, arranged in clusters like "medusoid" and delineated by the specifically stained membrane is found, or membrane-delineated polypoid organism are found at the surface of desquamated epithelial cells under the fluorescence microscope, a diagnosis of the presence of M. pneumoniae in the sputum can be made.

## Example D

An alternative path to obtain the specific membrane antibodies of mycoplasmas is to use the continuous cultures of fused cells, i.e., the hybridoma technique, first introduced by Köhler, G. and Milstein, C. (Nature 256:495-497, 1974) and subsequently modified by Buck, D. W., Kennett, R. H. and McGarrity, G. (In Vitro 18:377-381, 1981). An example is illustrated as follows:

### Preparation of organisms

A 5-day old liquid culture of medusoid colonies of M. pneumoniae is gently washed five times in PBS, each time being centrifuged at 2,500-3,000 rpm for 30 minutes. The final suspension is adjusted to $10^6$ medusoid colonies shown to be rich in membrane antigen by an indirect fluorescent antibody technique as described above.

### Immunization Protocol

Male BALB/c mice, 8 to 10 months old, are each injected with 1 ml of mycoplasma suspension intraperitoneally on days 1, 12 and 21.

### Hybridization (cell fusion) and hybridoma cell selection

Three days after the last immunization, the mice are killed, and three spleens are combined in complete Dulbecco's modified Eagle's medium without serum (DMEM) (purchased from GIBCO Laboratories, Grand Island, NY). The spleens are teased apart by sterile curved forceps and the single-cell suspension is washed in DMEM.

In order to fuse the spleen lymphocytes with the mouse myeloma cells (NSO cells), these two cells types are first suspended in a 50 ml centrifuge tube and mixed at a ratio of 5:1 ($10^8$ spleen cells and $2 \times 10^7$ NSO cells). After centrifugation at 1,000 rpm for 10 minutes the cell pellet is resuspended in 2 ml of prewarmed (37°C) polyethylene glycol (41% in DMEM), and incubated at 37°C for 1 minute. About 35 ml of prewarmed DMEM is added dropwise to the cell suspension to dilute the polyethylene glycol. The supernatant is removed after centrifugation, and the cell pellet is resuspended in 15 ml of prewarmed DMEM-HAT (complete DMEM containing 15% heat-inactivated horse serum, hypoxanthine $10^{-6}M$, aminopterin $4 \times 10^{-9}M$ and thymidine $1.6 \times 10^{-7}M$), and adjusted to a concentration of about $3 \times 10^5$ viable NSO myeloma cells/ml in DMEM-HAT. The final cell suspension is distributed in 0.1 ml/aliquots in the wells of 96- wells flat-bottom microculture plates. The plates are incubated at 37°C in 8% $CO_2$ in a humid incubator.

Growth of hybrid cells is indicated by change of color to yellow in the culture, usually 10-14 days following fusion. An aliquot of the supernatants is removed for screening membrane antibody activity by the indirect fluorescent antibody technique using glutaraldehyde-fixed medusoid colonies as antigen and FITC-labelled rabbit anti-mouse IgG as the second antibody. The medium in the wells containing cell culture producing specific membrane antibodies is then replaced by DMEM with horse serum, hypoxanthine and thymidine (without aminopterin). Regular DMEM horse serum medium is used after 10 days and the production of membrane antibodies of M. pneumoniae is continuously monitored by indirected fluorescent antibody assays.

The culture is finally cloned by diluting the cells with DMEM horse serum containing 0.2% agar so that after incubation isolated cell colonies appear in a thin layer of semisolid medium in Petri dishes. The single cell colonies are picked up by capillary Pasteur pipettes and transferred to wells each containing 0.1 ml new medium for multiplication. The pure hybridoma cell culture in each well is screened for membrane antibodies after 10-14 days incubation.

For large scale production, the described antibodies can be produced either by propagating the hybridoma cells in the peritoneal cavity of BALB/c mice and the antibodies harvested from ascites, or the described antibodies can be produced in tissue culture flask, preferably in serum free medium. If the antibody titer is too low, the antibody can be concentrated by ultrafiltration.

Example E

To illustrate the cross reactivity between the membrane antigen of Mycoplasma pneumoniae and that of Mycoplasma genitalium, the medusoid colonies of these two organisms are fixed in 1% glutaraldehyde and allowed to react with convalescent sera of M. pneumoniae patients or with immune rabbit sera raised by live medusoid colonies of M. pneumoniae. The antigen antibody reaction is demonstrated by indirect fluorescent antibody technique. The results show a similar binding pattern of specific IgG and IgM human and rabbit antibodies by the membranes of M. pneumoniae and M. genitalium. Other mycoplasma species, such as Mycoplasma hominis, Mycoplasma orale and Mycoplasma salivarium, do not react with these antibodies. This example shows the potential of using M. pneumoniae medusoid antigens to detect the presence of antibodies in patients with M. genitalium infection, and using the specific membrane monoclonal antibodies of Mycoplasma pneumoniae to detect the membrane of Mycoplasma genitalium in the excretions of the urethra.

It should be noted that the above described procedures are also applicable to other mycoplasma infections.

While the invention has now been described in what are considered to be the preferred embodiments, it should be understood that the invention is not limited to the disclosed embodiments but, is intended to cover various modifications and equivalent processes included within the spirit and scope of the appended claims, which claim scope is to be accorded the broadest interpretation so as to encompass all such modifications and equivalent processes.

**Claims**

1. A mycoplasma grown in liquid medium, characterised in that it is predominantly in the form of medusoid microcolonies comprising polyps or vesicles 1–10 μm in diameter against which antibodies to one or more specific membrane antigens of said mycoplasma can be raised.

2. A mycoplasma as claimed in claim 1 of the species M. pneumoniae or M. genitalium.

3. A mycoplasma as claimed in claim 1 or claim 2 fixed in an aliphatic aldehyde.

4. A mycoplasma as claimed in claim 3 fixed in glutaraldehyde.

5. A method for preparing a mycoplasma predominantly in the form of medusoid microcolonies as claimed in claim 1 comprising:

   a) forming a culture of said mycoplasma in a dialysed, particle free liquid medium, and incubating said culture therein;

   b) transferring a resulting portion of said incubating culture to a fresh batch of said liquid medium, and again incubating the same therein to form a subculture;

   c) repeating step b) at least once to provide at least 3 total incubations and a final subculture;

   d) incubating said final subculture for a period of time sufficient to form tuberous colonies of mycoplasma of about 25–50 μm diameter or conglomerates of said colonies;

   e) separating said tuberous colonies and reincubating the same in successive fresh batches of medium until floating colonies of mycoplasma are the predominent growth form;

   (f) harvesting said tuberous floating colonies, homogenizing the same, and re-incubating in fresh medium to form round or ovoid microcolonies of from 5 to 25 µm diameter or conglomerates of said microcolonies; and

(g) harvesting and washing the microcolonies thus obtained.

6. A method as claimed in claim 5 wherein said harvesting of the microcolonies in step (g) is achieved by centrifugation at about 2,500-3,000 rpm (700 - 1,000 xg).

7. A method as claimed in claim 5 or claim 6 wherein said liquid medium comprises (1) a sterilized dialysate of broth, yeast extract, thallium acetate and distilled water, (2) gamma-gobulin-free horse serum, and (3) penicillin and is adjusted to a pH of 7.0 - 7.8.

8. A method of detecting antibodies against one or more membrane antigens of a mycoplasma in liquid sample comprising the steps of:

(a) contacting said sample with a sample of a mycoplasm predominantly in the form of medusoid microcolonies as claimed in any one of claims 1 to 4;

(b) washing off the unbound fraction of the said liquid sample;

(c) treating the residue with fluorescent - labelled antibodies capable of binding antibodies of the species from which said liquid sample was derived;

(d) washing off the unbound fluorescent antibodies; and

(e) examining said thus prepared and reacted microcolonies to determine the membrane fluorescence characteristics thereof.

9. A method for obtaining antibodies against one or more specific membrane antigens of a mycoplasma comprising the steps of:

a) isolating animal immunoglobulins from an animal previously immunised against a live mycoplasma predominantly in the form of medusoid microcolonies as claimed in claim 1 or claim 2;

b) removing antibodies non-specific for a specific membrane antigen of said mycoplasma by absorbing on acetone-fixed colonies of a mycoplasma culture.

10. A method as claimed in claim 9 wherein in step (c) washed acetone-fixed tuberous colonies from a 4 weeks old culture are employed.

11. Use of a mycoplasma predominantly in the form of medusoid microcolonies as claimed in any one of claims 1 to 4 to prepare a monoclonal antibody to a specific membrane antigen of said mycoplasma.

12. A method of detecting one or more specific membrane antigens of a mycoplasma in sputum or another exudate of a patient wherein one or more antibodies prepared as in any one of claims 9 to 11 are employed.

13. A method for preparing a mycoplasma predominantly in the form of tuberous floating colonies comprising steps (a) to (e) as defined in claim 5.

**Revendications**

1. Mycoplasme cultivé dans un milieu liquide, caractérisé en ce qu'il se présente, de façon prédominante, sous la forme de microcolonies médusoïdes comprenant des polypes ou des vésicules d'un diamètre de 1 à 10 µm, vis-à-vis duquel des anticorps d'un ou plusieurs antigènes spécifiques de membranes du mycoplasme précité peuvent être élevés.

2. Mycoplasme suivant la revendication 1 de l'espèce M. pneumoniae ou M. genitalium.

3. Mycoplasme suivant la revendication 1 ou la revendication 2, fixé dans un aldéhyde aliphatique.

4. Mycoplasme suivant la revendication 3, fixé dans le glutaraldéhyde.

5. Procédé de préparation d'un mycoplasme se présentant, de manière prédominante, sous la forme de microcolonies médusoïdes, suivant la revendication 1, caractérisé en ce que:

a) on forme une culture du mycoplasme précité dans un milieu liquide, dialysé, exempt de particules et on y incube ladite culture,

b) on transfère une fraction résultante de ladite culture en incubation dans un lot frais du milieu liquide précité et on y incube à nouveau celui-ci pour former une sub-culture,

c) on répète l'étape b) au moins une fois de façon à parvenir à au moins 3 incubations totales et à une subculture finale,

d) on incube ladite sub-culture finale pendant une période qui suffit à former des colonies tubéreuses de mycoplasme d'un diamètre d'environ 25– 50 µm, ou des conglomérats des colonies précitées,

e) on sépare lesdites colonies tubéreuses et on réincube celles-ci dans des lots frais successifs de milieu jusqu'à ce que des colonies tubéreuses flottantes de mycoplasme constituent la forme de croissance prédominante,

f) on récolte lesdites colonies flottantes tubéreuses, on les homogénéise et on les ré-incube dans du milieu frais pour former des microcolonies rondes ou ovoïdes d'un diamètre de 5 à 25 µm, ou des conglomérats des microcolonies précitées et

g) on récolte et lave les microcolonies ainsi obtenues.

6. Procédé suivant la revendication 5, caractérisé en ce que la récolte des microcolonies de l'étape g) est achevée par centrifugation à environ 2500– 3000 tpm (700–1000 xg).

7. Procédé suivant la revendication 5 ou la revendication 6, caractérisé en ce que le milieu liquide comprend (1) un dialysat stérilisé de bouillon, d'extrait de levure, d'acétate de thallium et d'eau distillée, (2) un sérum de cheval exempt de gammaglobuline et (3) de la pénicilline et son pH est ajusté à une valeur de 7,0 à 7,8.

8. Procédé de détection d'anticorps vis-à-vis d'un ou plusieurs antigènes de membranes de mycoplasmes dans un échantillon liquide, caractérisé en ce qu'il comprend les étapes consistant à:

a) mettre en contact l'échantillon précité avec un échantillon d'un mycoplasme se présentant, de manière prédominante, sous la forme de microcolonies médusoïdes suivant l'une quelconque des revendications 1 à 4,

b) laver la fraction non liée de l'échantillon liquide précité,

c) traiter le résidu par des anticorps marqués de manière à être fluorescents, capables de lier des anticorps de l'espèce dont l'échantillon de liquide précité fut dérivé,

d) éliminer par lavage les anticorps fluorescents non liés et

e) examiner les microcolonies ainsi préparées et ayant réagi pour en déterminer les caractéristiques de fluorescence des membranes.

9. Procédé d'obtention d'anticorps vis-à-vis d'un ou plusieurs antigènes spécifiques ·de membranes de mycoplasmes, caractérisé en ce qu'il comprend les étapes consistant à:

a) isoler des immunoglobulines animales d'un animal préalablement immunisé contre un mycoplasme vivant se présentant, de manière prédominante, sous la forme de microcolonies médusoïdes suivant la revendication 1 ou la revendication 2,

b) éliminer les anticorps aspécifiques d'un antigène de membrane spécifique du mycoplasme par absorption d'une culture de mycoplasme sur des colonies fixées à l'acétone.

10. Procédé suivant la revendication 9, caractérisé en ce que, dans l'étape b), on utilise des colonies tubéreuses, fixées à l'acétone, lavées, d'une culture vieille de 4 semaines.

11. Utilisation d'un mycoplasme se présentant, de manière prédominante, sous la forme de microcolonies médusoïdes suivant l'une quelconque des revendications 1 à 4, en vue de préparer un anticorps monoclonal d'un antigène spécifique de membrane du mycoplasme précité.

12. Procédé de détection d'un ou plusieurs antigènes spécifiques de membranes de mycoplasmes dans les crachats ou tout autre exsudat d'un patient, caractérisé en ce que l'on utilise un ou plusieurs anticorps préparés de la manière décrite dans l'une quelconque des revendications 9 à 11.

13. Procédé de préparation d'un mycoplasme se présentant, de façon prédominante, sous la forme de colonies flottantes, tubéreuses, comprenant les étapes a) à e) telles que définies dans la revendication 5.

**Patentansprüche**

1. In flüssigem Medium gezüchtetes Mycoplasma, dadurch gekennzeichnet, daß es hauptsächlich in der Form von medusenartigen Mikrokolonien vorliegt, die Polypen oder Vesikel von 1–10 µm Durchmesser enthalten, gegen welche Antikörper gegen ein oder mehrere spezifische Membranproteine des Mycoplasmas hergestellt werden können.

2. Mycoplasma nach Anspruch 1 der Spezies M. pneumoniae oder M. genitalium.

3. Mycoplasma nach Anspruch 1 oder Anspruch 2 fixiert in einem aliphatischen Aldehyd.

4. Mycoplasma nach Anspruch 3, fixiert in Glutaraldehyd.

5. Verfahren zur Herstellung eines Mycoplasmas hauptsächlich in der Form von medusenartigen Mikrokolonien nach Anspruch 1, dadurch gekennzeichnet, daß man

a) eine Kultur des Mycoplasmas in einem dialysierten, partikelfreien flüssigen Medium bildet und die Kultur in diesem inkubiert,

b) einen resultierenden Teil der inkubierten Kultur in einen frischen Batch des flüssigen Mediums transferiert und diesen wiederum darin inkubiert, um eine Subkultur zu bilden,

c) Schritt b) mindestens einmal wiederholt, um mindestens einmal wiederholt, um mindestens drei Gesamt-Inkubationen und eine endgültige Subkultur zu schaffen,

d) die endgültige Subkultur über einen Zeitraum inkubiert, der ausreicht, um tuberöse Mycoplasmakolonien mit einem Durchmesser von etwa 25–50 µm oder Konglomerate dieser Kolonien zu bilden,

e) die tuberösen Kolonien abtrennt un diese in nachfolgenden frischen Medium-Batches reinkubiert, bis die überwiegende Wachstumsform durch tuberöse, schwebende Mycoplasmakolonien gebildet wird,

f) die tuberösen schwebenden Kolonien erntet, homogenisiert und in frischem Medium reinkubiert, um runde oder ovale Mikrokolonien mit einem Durchmesser von 5–25 µm oder Konglomerate dieser Mikrokolonien zu bilden und

g) die so erhaltenen Mikrokolonien erntet und wäscht.

6. Verfahren nach Anspruch 5, dadurch gekennzeichnet, daß man die Mikrokolonien in Schritt g) durch Zentrifugieren bei etwa 2500–3000 UpM (700–1000 xg) ernte.

7. Verfahren nach den Ansprüchen 5 oder Anspruch 6, dadurch gekennzeichnet, daß das flüssige Medium (1) ein sterilisiertes Dialysat von Brühe, Hefeextrakt, Thalliumacetat und destilliertem Wasser, (2) Gammaglobulin freies Pferdeserum und (3) Penicillin enthält und auf einen pH-Wert von 7,0 bis 7,8 eingestellt ist.

8. Verfahren zum Nachweis von Antikörpern gegen ein oder mehrere Antigene eines Mycoplasmas in einer Flüssigprobe, dadurch gekennzeichnet, daß man

a) die Probe mit einer Probe eines Mycoplasmas in Kontakt bringt, welches überwiegend in der Form von medusenartigen Mikrokolonien nach den Ansprüchen 1 bis 4 vorliegt,

b) die nicht-gebundene Fraktion der flüssigen Probe abspült,

c) den Rückstand mit Fluoreszenz-markierten Antikörpern behandelt, die in der Lage sind, Antikörper derjenigen Spezies zu binden, von der die flüssige Probe stammt,

d) die nicht-gebundenen fluoreszierenden Antikörper abspült und

e) die so behandelten und umgesetzten Mikrokolonien untersucht, um ihre Membranfluoreszenz-Eigenschaften zu bestimmen.

9. Verfahren zum Herstellen von Antikörpern gegen ein oder mehrere spezifische Membranantigene eines Mycoplasmas, dadurch gekennzeichnet, daß man

a) tierische Immunglobuline aus einem Tier isoliert, welches zuvor gegen ein lebendes Mycoplasma immunisiert wurde, welches überwiegend in der Form von medusenartigen Mikrokolonien nach Anspruch 1 oder Anspruch 2 vorliegt und

b) Antikörper, die nicht spezifisch für ein spezifisches Membranantigen des Mycoplasmas sind, durch Absorbieren auf Aceton-fixierte Kolonien einer Mycoplasma-Kultur entfernt.

10. Verfahren nach Anspruch 9, dadurch gekennzeichnet, daß man in Stufe b) gewaschene, Aceton-fixierte tuberöse Kolonien aus einer vier Wochen alten Kultur verwendet.

11. Verwendung eines Mycoplasmas, welches überwiegend in der Form von medusenartigen Mikrokolonien nach jeglichem der Ansprüche 1 bis 4 vorliegt, zur Herstellung von monoklonalen Antikörpern gegen ein spezifisches Membranantigen des Mycoplasmas.

12. Verfahren zum Nachweis eines oder mehrerer spezifischen Membranantigene eines Mycoplasmas in sputum oder einer anderen Ausscheidung eines Patienten, bei dem ein oder mehrere Antikörper hergestellt nach jeglichem der Ansprüche 9 bis 11 verwendet werden.

13. Verfahren zur Herstellung eines Mycoplasmas, welches überwiegend in der Form von tuberösen schwebenden Kolonien vorliegt, gekennzeichnet durch die Schrite a) bis e) nach Anspruch 5.